# FASCICULE DE BREVET EUROPEEN

(11) **EP 0 327 411 B1**
(45) Date de publication et mention de la délivrance du brevet: **27.04.1994**
(21) Numéro de dépôt: 89400026.4
(22) Date de dépôt: 04.01.1989
(51) Int. Cl.: C08G 69/10, A61L 15/16, A61L 15/26, A61L 25/00

(54) **Procédé de préparation d'un copolymère de deux acides alpha-aminés**
Verfahren zur Herstellung eines Copolymers aus zwei alpha-Aminsäuren
Process for the preparation of a copolymer from two alpha-amino acids

(30) Priorité: 06.01.1988 FR 8800064
(43) Date de publication de la demande: 09.08.1989
(73) Titulaire: SYNTHELABO, 92350 Le Plessis Robinson (FR)
(72) Inventeur: Brack, André, F-45160 Olivet (FR)
(74) Mandataire: Kedinger, Jean-Paul

(56) Documents cités:
- CHEMICAL ABSTRACTS, vol. 63, no. 1, 1965, colonne 705f-g, Columbus, Ohio, US; & JP-A-20 527
- DIE ANGEWANDTE MAKROMOLEKULARE CHEMIE, vol. 40/41, no. 580, 1974, pages 493-506; G. EBERT et al.: "Fasern und Folien aus Poly-alpha-Aminosäuren"
- MAKROMOLEKULARE CHEMIE, vol. 179, no. 12, décembre 1978, pages 2829-2835; A. BRACK et al.: "Polycondensation d'acides alpha-aminés par des systèmes iode-phosphites en présence de pyridine ou d'imidazole"

## Description

La présente invention concerne un nouveau procédé de synthèse d'un copolymère de deux acides α-aminés, qui comprend la préparation d'un mélange du N-carboxyanhydride d'un premier acide α-aminé et du N-carboxyanhydride d'un second acide α-aminé, suivie de la copolymérisation de ce mélange. Elle a également pour objet une nouvelle application du copolymère obtenu par ce procédé.

Conformément aux procédés connus de ce type, on obtient le mélange des N-carboxyanhydrides des premier et second acides α-aminés (i) en préparant séparément le N-carboxyanhydride du premier acide α-aminé et le N-carboxyanhydride du second acide α-aminé, par réaction séparée du phosgène sur le premier acide aminé et sur le second acide aminé, puis (ii) en mélangeant, après éventuelle purification, les deux N-carboxyanhydrides ainsi obtenus. Du fait de la préparation distincte des deux N-carboxyanhydrides, ces procédés connus nécessitent la mise en oeuvre de deux chaînes parallèles de production comprenant chacune un réacteur muni d'accessoires divers (agitateur, moyens de mesure de la température, dispositif de reflux, amenées pour les réactifs, moyens de règlage du débit de phosgène, moyens d'évacuation du mélange réactionnelle), de moyens de purification du N-carboxyanhydride et de moyens de transfert dudit N-carboxyanhydride vers le réacteur de polymérisation. La mise en oeuvre de ces procédés fait donc nécessairement appel à un équipement complexe et, partant, coûteux. En outre, il est souvent difficile de programmer toutes les opérations effectuées avec cet équipement pour qu'il y ait synchronisme entre les deux chaînes parallèles de production. Une telle difficulté résulte de la complexité des équipements utilisés, mais aussi du fait que la réaction de l'un des acides aminés avec le phosgène peut nécessiter une durée plus importante que l'autre acide aminé. Il s'ensuit qu'il arrive fréquemment, avec les procédés connus décrits ci-dessus, que les opérations relatives à l'un des N-carboxyanhydrides soient terminées avant celles relatives à l'autre N-carboxyanhydride de sorte que le N-carboxyanhydride obtenu en premier doit être stocké pendant une période plus ou moins longue avant d'être utilisé en vue de la copolymérisation. Or, il a été constaté que ces N-carboxyanhydrides se conservent mal et sont sensibles à divers agents, notamment l'humidité, ce qui oblige à prendre des précautions qui compliquent encore les equipements et la synthèse desdits N-carboxyanhydrides.

Par ailleurs, par G. Ebert et al., Angew. Makromol. Chem. 40/41 (1974), on connait des copolymères de leucine et d'acide glutamique et des copolymères de lysine et d'alanine.

La présente invention a pour but de surmonter les inconvénients susmentionnés et pour ce faire elle propose un procédé conforme à celui défini au premier paragraphe de cette description, qui se caractérise en ce que le mélange des N-carboxyanhydrides des premier et second acides α-aminés est obtenu par action du, phosgène sur un mélange d'un premier et d'un second acides α-aminés ayant sensiblement la même vitesse de réaction avec le phosgène. On comprendra en effet qu'en sélectionnant de manière appropriée les acides aminés de départ, à savoir ceux ayant sensiblement la même vitesse de réaction avec le phosgène, il devient possible de les faire réagir simultanément avec le phosgène dans un réacteur unique et une seule chaîne de production est dès lors suffisante pour obtenir les N-carboxyanhydrides désirés ce qui non seulement simplifie grandement l'équipement à mettre en oeuvre pour la synthèse du copolymère désiré mais encore évite tout risque de dégradation des N-carboxyanhydrides puisque ces derniers sont obtenus simultanément et le stockage temporaire de l'un ou de l'autre n'est plus nécessaire.

Les premier et second acides α-aminés sont constitués par la leucine, notamment la L-leucine, et par l'acide glutamique ou l'acide glutamique dont la fonction carboxyle la plus éloignée de la fonction amine est estérifié par un groupe méthyle ou benzyle, notamment l'acide L-glutamique ou l'ester méthylique ou benzylique de l'acide L-glutamique.

Le mélange des premier et second acides α-aminés destiné à réagir avec le phosgène comprend avantageusement de 40 à 60% en mole de l'un de ces acides aminés et 60 à 40% en mole de l'autre.

La réaction des acides α-aminés peut être effectuée dans un solvant organique et on peut citer le dioxane ou le tétrahydrofurane à titre d'exemples.

Avant de procéder a la copolymérisation des N-carboxyanhydrides résultants, il est souhaitable de les soumettre à un traitement approprié pour réduire leur teneur en dérivés chlorés, de préférence jusqu'à une valeur inférieure à 0,1%, ces dérivés étant produits au cours de la réaction du phosgène avec les acides α-aminés. Un tel traitement est décrit ci-après dans le cadre de préparations données à des fins d'illustration de l'invention.

Le mélange des N-carboxyanhydrides est ensuite soumis à la réaction de copolymérisation, avantageusement à des températures modérées, dans un solvant approprié tel que le dioxane et en présence d'un initiateur de copolymérisation comme une amine tertiaire (triéthylamine, tributylamine, triéthanolamine, etc.) éventuellement en présence d'un sel de lithium (chlorure ou nitrate par exemple) ou une base forte telle qu'un alcoolate de métal alcalin (méthylate de sodium par exemple). Le rapport de la somme du nombre de moles des N-carboxyanhydrides au nombre de moles de l'initiateur de copolymérisation est fonction de l'initiateur utilisé et de la viscosité souhaitée pour le copolymère. Ainsi, dans le cas où l'initiateur est constitué par la tributylamine, un rapport situé dans l'intervalle allant de 25 à 200 conduit à l'obtention d'un copolymère possédant une viscositè intrinséque appropriée aux usages décrits ci-après auxquels est destiné ce copolymère. De même, lorsque l'initiateur est constitué par le méthylate de sodium, un rapport situé dans l'intervalle allant de 100 à 1000 conduit à l'obtention d'un copolymère approprié.

Le copolymère obtenu par mise en oeuvre du procédé décrit ci-dessus, est isolé en coulant le mélange réactionnel dans un grand excès d'eau. En procédant ainsi, le copolymère précipite sous la forme d'un produit fibreux très hydrophobe et très volumineux.

Ce copolymère trouve essentiellement son application comme moyen de revètement des plaies cutanées, des zones cutanées en cours de réparation, des brulûres et des zones de prélèvement de greffes, moyen de revêtement qui favorise la cicatrisation tout en assurant, jusqu'à la guérison ou la cicatrisation, la protection de ces plaies et zones contre les agressions extérieures, que ces dernières soient d'ordre mécanique ou microbien.

En vue de cette utilisation, le copolymère est amené sous une forme appropriée à son dépôt en mince couche sur la partie a protèger. Ainsi, ce copolymère peut par exemple être amené sous la forme d'un film mince ou d'une poudre ou incorporé dans un gel. A partir de ce copolymère on peut notamment réaliser un film mince par la technique bien connue de l'inversion de phase comme cela sera décrit ci-après avec davantage de détails. Un tel film est généralement translucide, souple et non poreux, il présente une épaisseur de préférence comprise entre 300 et 750µm et il est perméable à l'oxygène, à la vapeur d'eau et à certaines solutions désinfectantes que l'on pourrait éventuellement désirer appliquer à travers ce film. En vue de l'obtention d'un film ayant ces caractéristiques, il est souhaitable que le copolymère ait une viscosité intrinsèque à 30°C dans l'acide dichloroacétique comprise entre 0,5 et 3 dl/g. Après guérison ou cicatrisation, le film, la poudre ou le gel est retiré ou s'élimine spontanément de la zone sur laquelle il a été appliqué.

Les préparations suivantes sont données a titre d'illustration de l'invention.

### Préparation du L-glutamate de méthyle [L-Glu(OMe)]

Dans un réacteur de 2 litres, on place 1,25 litre de méthanol. On coule ensuite à une température inférieure à 20°C, 100ml de chlorure d'acétyle. La coulée terminée et dans les mêmes conditions de température on ajoute en une seule fois 148 g d'acide L-glutamique et on agite à température ambiante pendant 24 heures On coule enuite dans le mélange obtenu 125 ml de pyridine à une température inférieure à 20°C. Il se forme un précipité et on agite encore pendant 48 heures à température ambiante. On sépare le précipité par filtration, on le lave 2 fois à l'éthanol et une fois à l'éther, puis le sèche sous vide ce qui conduit à 174 g (rendement : 86,56%) de l'ester attendu qui se présente sous la forme d'un produit blanc.

| Analyse élémentaire | |
|---|---|
| Trouvé (%) | Calculé (%) |
| C:45,22-45,23 | 44,72 |
| H:7,14-7,16 | 6,88 |
| N:8,46-8,46 | 8,69 |

CCM : éluant : BuOH/CH₃COOH/H₂O : 4/1/1

### Préparation du mélange des N-carboxyanhydrides de la L-leucine et du L-glutamate de méthyle

Dans un réacteur de 2 litres muni d'un système anti-retour de 2 litres, d'une arrivée de gaz permettant un débit important de phosgène et d'argon, d'un réfrigérant à boules, d'un receveur à soude et d'une colonne à charbon actif, on place 65,5 g de L-leucine (0,5 mole), 80,5 g de L-glutamate de méthyle (0,5 mole) et 1600 ml de dioxane anhydre passé préalablement sur alumine pour le débarrasser des peroxydes qu'il peut contenir.

La suspension obtenue est purgée à l'argon pendant 30 mn. On passe ensuite un courant rapide de phosgène tout en agitant énergiquement le mélange. On porte la température à l'intérieur du ballon a 40°C. Au bout de 6 heures, le mélange réactionnel devient homogène. La solution est ensuite balayée à l'argon pendant au moins 48 heures à 40°C dans le but d'éliminer le maximum de dérivés chlorés dissous dans la solution. On évapore sous vide et épuise le résidu avec 20 litres de dioxane anhydre par portions de 2 litres. On vérifie dans le distillat que la quantité de dérivés chlorés diminue progressivement.

La solution dans le dioxane est évaporée sous vide et le résidu est repris avec 800 ml d'hexane sec. Il y a cristallisation d'un produit sur lequel on effectue, après sèchage sous vide, un dosage potentiomètrique des ions chlorure. Dans le cas où la teneur en ions chlorure est supérieure a 0,4%, il y a lieu de reprendre les épuisements au dioxane. Dans le cas contraire, un traitement final avec la quantité théorique de Ag₂O en solution dans le dioxane doit permettre d'abaisser la teneur en ions chlorure a moins de 0,1%. C'est la condition nécessaire pour obtenir à l'étape suivante un copolymère convenable pour la réalisation d'un film mince. On obtient finalement environ 140 g (rendement : 81,39%) du mélange attendu prêt pour la copolymérisation.

### Préparation du copolymère de la L-leucine et du L-glutamate de méthyle

Dans un réacteur de 2 litres muni d'un agitateur puissant, on introduit 137 g du mélange préparé comme indiqué ci-dessus et 2000 ml de dioxane anhydre. La solution obtenue est portée, sous argon, à une température de 40°C. On introduit alors en une seule fois 16 ml d'une solution 0,1 M de méthylate de sodium dans le dioxane ou 8 ml d'une solution 1 M de tributylamine dans le dioxane. Au bout de 4 heures d'agitation la réaction de polymérisation démarre et la solution devient de plus en plus visqueuse. On laisse encore agiter pendant 3 jours en vérifiant la disparition de la bande caractéristique des N-carboxyanhydrides à 1785 cm⁻¹ en I.R.. Le temps de polymérisation dépend de la quantité de dérivés chlorés présente dans le milieu et de la quantité d'initiateur de polymérisation utilisé. Pour une teneur en dérivés chlorés inférieure à 0,1%, un rapport en moles du mélange des N-carboxyanhydrides à l'initiateur de 100 dans le cas de l'utilisation de la tributylamine ou de 500 dans le cas de l'utilisation du mèthylate de sodium est particulièrement convenable pour obtenir un copolymère apte à être mis sous la forme d'un film.

La solution visqueuse du copolymère dans le dioxane est ensuite coulée lentement dans un récipient contenant 10 litres d'eau. Le copolymère précipite au contact de l'eau, on sépare le précipité par filtration, on le sèche à l'air sur papier et termine le sèchage sous vide. On obtient ainsi 85 g d'un produit blanc ayant les caractéristiques suivantes :
. viscosité intrinséque a 30°C dans l'acide dichloroacétique : 1,79 dl/g
. analyse élémentaire :

| | Trouvé (%) | Calculé (%) |
|---|---|---|
| C | 56,04-56,17 | 56,23 |
| N | 7,99-7,96 | 7,83 |
| N | 10,48-10,42 | 10,93 |

(Les pourcentages calculés sont déterminés pour une quantité équimolaire de L-leucine et de L-glutamate de méthyle).
. pouvoir rotatoire : α=-44° (c=1% dans l'acide dichloroacétique) à 20°C pour la raie D du sodium.
. Chromatographie liquide haute pression : dosage de la L-leucine et de l'acide L-glutamique après une hydrolyse de 48 heures dans HCl concentré à 100°C :
L-leucine : 44%
acide L-glutamique : 56%

### Fabrication d'un film mince à partir du copolymère de L-leucine et du L-glutamate de méthyle

On commence par réaliser un collodion du copolymère dans un solvant organique très polaire tel que la N-mèthyl pyrrolidone, la N-diméthylacétamide, la N-diméthylformamide, le diméthylsulfoxyde ou l'acide difluoro- ou trifluoro acétique. Après coulée, sur l'épaisseur voulue (500µm par exemple), du collodion obtenu, sur une plaque en verre, on immerge la plaque supportant le copolymère dans plusieurs bains d'eau successifs pour coaguler le collodion et laver le film résultant. On détache ce film de la plaque de verre et on l'immerge dans un bain contenant 75% de PEG 600 et 25% de sérum physiologique pour en imprégner le film en vue de sa conservation. Après mise en sachet imperméable, on stérilise le tout par irradiation de rayonnement γ.

## Revendications

1. Procédé de synthèse d'un copolymère de deux acides α-aminés, qui comprend la préparation d'un mélange du N-carboxyanhydride d'un premier acide a-aminé et du N-carboxyanhydride d'un second acide α-aminé, suivie de la copolymérisation de ce mélange, caractérisé en ce que ledit mélange est obtenu par action du phosgène sur un mélange d'un premier acide α-aminé constitué par la leucine et d'un second acide α-aminé constitué par l'acide glutamique ou l'acide glutamique dont la fonction carboxyle la plus éloignée de la fonction amine est estérifiée par un groupe méthyle ou benzyle.

2. Procédé selon la revendication 1, caractérisé en ce qu'avant la copolymérisation dudit mélange, ce dernier est traité, si besoin est, pour réduire sa teneur en dérivés chlorés jusqu'à une valeur inférieure à 0,1%.

3. Procédé selon la revendication 2, caractérisé en ce que ledit traitement comprend la réaction dudit mélange avec Ag₂O.

4. Procédé selon l'une des revendications précédentes, caractérisé en ce que ledit mélange des premier et second acides α-aminés comprend de 40 à 60 % en mole de l'un de ces acides aminés et 60 à 40 % en mole de l'autre.

5. Agent pour le revêtement des plaies cutanées, des zones cutanées en cours de réparation, des brûlures et des zones de prélèvement de greffes, caractérisé en ce qu'il comprend le copolymère obtenu par la mise en oeuvre du procédé selon l'une quelconque des revendications 1 à 4.

6. Agent selon la revendication 5, caractérisé en ce qu'il se présente sous la forme d'un film ayant une épaisseur comprise entre 300 et 750 µm, d'une poudre ou d'un gel.

## Claims

1. Process of synthesizing a copolymer of two α-amino acids which comprises the preparation of a mixture of the N-carboxyanhydride of a first α-amino acid and the N-carboxyanhydride of a second α-amino acid, followed by the copolymerization of this mixture, characterized in that said mixture is obtained by action of phosgene on a mixture of a first amino acid constituted by leucine and a second α-amino acid constituted by glutamic acid or glutamic acid whose carboxyl function the furthest away from the amine function is esterified by a methyl or benzyl group.

2. Process according to claim 1; characterized in that before the copolymerization of said mixture, if necessary, the latter is subjected to a treatment for reducing its chlorinated derivative content to a value less than 0.1 %.

3. Process according to claim 2, characterized in that said treatment comprises the reaction of said mixture with Ag₂O.

4. Process according to one of the preceding claims, characterized in that said mixture of the first and second α-amino acids comprises from 40 to 60 mole % of one of these amino acids and 60 to 40 mole % of the other.

5. Agent for covering skin wounds, skin zones being repaired, bums and zones from which grafts have been taken, characterized in that it comprises the copolymer obtained by the process according to any one of claims 1 to 4.

6. Agent according to claim 5, characterized in that it is in the form of a film having a thickness comprised between 300 and 750 µm, a powder or a gel.

## Patentansprüche

1. Verfahren zur Synthese eines Copolymers aus zwei α-Aminosäuren, welches die Herstellung eines Gemisches aus dem N-Carbonsäureanhydrid einer ersten α-Aminosäure und aus dem N-Carbonsäureanhydrid einer zweiten α-Aminosäure, gefolgt von der Copolymerisation des Gemisches umfaßt, **dadurch gekennzeichnet**, daß das Gemisch durch Einwirken von Phosgen auf ein Gemisch aus einer ersten α-Aminosäure, welche aus Leucin besteht. und einer zweiten α-Aminosäure, welche aus Glutaminsäure oder Glutaminsäure, deren von der Aminfunktion am weitesten entfernte Carboxylfunktion mit einer Methylgruppe oder Benzylgruppe verestert ist, besteht, erhalten wird.

2. Verfahren nach Anspruch 1, **dadurch gekennzeichnet**, daß vor der Copolymerisation des Gemisches letzteres, sofern dies erforderlich ist, behandelt wird, um seinen Gehalt an chlorierten Derivaten bis auf einen Wert unterhalb von 0,1% zu verringern.

3. Verfahren nach Anspruch 2, **dadurch gekennzeichnet**, daß die Behandlung die Reaktion des Gemisches mit Ag₂O umfaßt.

4. Verfahren nach einem der vorhergehenden Ansprüche, **dadurch gekennzeichnet**, daß das Gemisch aus der ersten und der zweiten α-Aminosäure 40 bis 60 Mol-% von einer dieser Aminosäuren und 60 bis 40 Mol-% von der anderen umfaßt.

5. Mittel zum Bedecken von Hautwunden, von verheilenden Hautbereichen, von Brandwunden und von Bereichen, wo Transplantate entnommen wurden, **dadurch gekennzeichnet**, daß es das Copolymer umfaßt, welches durch Ausführen des Verfahrens nach einem der Ansprüche 1 bis 4 erhalten wird.

6. Mittel nach Anspruch 5, **dadurch gekennzeichnet**, daß es in Form eines Films mit einer Dicke zwischen 300 und 750 µm, eines Pulvers oder eines Gels vorliegt.
